(19) Europäisches Patentamt
European Patent Office
Office européen des brevets

(11) EP 1 486 196 B1

(12) # FASCICULE DE BREVET EUROPEEN

(45) Date de publication et mention de la délivrance du brevet:
**23.06.2010 Bulletin 2010/25**

(51) Int Cl.:
***A61Q 5/04*** *(2006.01)* ***A61K 8/46*** *(2006.01)*
***A61K 8/84*** *(2006.01)*

(21) Numéro de dépôt: **04291266.7**

(22) Date de dépôt: **18.05.2004**

(54) **Composition réductrice pour la déformation permanente des fibres kératiniques et procédé de déformation permanente**

Reduzierende Zusammensetzung zur dauerhaften Verformung von Keratinfasern und Verfahren zur dauerhaften Verformung

Reducing composition for permanently deforming keratin fibers and process for the permanent deformation

(84) Etats contractants désignés:
**AT BE BG CH CY CZ DE DK EE ES FI FR GB GR HU IE IT LI LU MC NL PL PT RO SE SI SK TR**

(30) Priorité: **19.05.2003 FR 0305971**

(43) Date de publication de la demande:
**15.12.2004 Bulletin 2004/51**

(73) Titulaire: **L'Oréal**
**75008 Paris (FR)**

(72) Inventeurs:
- **Fondin, Thomas**
  **95150 Taverny (FR)**
- **Sabbagh, Anne**
  **92500 Rueil Malmaison (FR)**

(74) Mandataire: **Casalonga, Axel**
**Casalonga & Partners**
**Bayerstrasse 71/73**
**80335 München (DE)**

(56) Documents cités:
**EP-A- 0 712 623** **EP-A- 0 723 772**
**DE-A- 19 840 190** **DE-A- 19 902 246**
**FR-A- 2 270 846** **FR-A- 2 514 640**
**FR-A- 2 548 019** **FR-A- 2 831 804**
**GB-A- 2 026 052** **US-A- 2 708 940**
**US-A- 4 381 919** **US-A- 5 700 455**
**US-A- 6 024 949**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention concerne une composition réductrice pour la déformation permanente des fibres kératiniques, et en particulier les cheveux, un procédé de déformation permanente mettant en oeuvre cette composition réductrice ainsi qu'un dispositif à compartiments ou kit.

Au sens de la présente invention, on entend par procédé de déformation permanente, tout procédé durable dans le temps, de mise en forme des cheveux ou de frisage.

En matière de déformation permanente des cheveux, il est connu de réaliser, en un premier temps, sur les cheveux préalablement mis sous tension à l'aide de rouleaux, bigoudis ou autres, l'ouverture des liaisons disulfures de la cystine de la kératine à l'aide d'une composition contenant un agent réducteur puis, généralement après un rinçage de la chevelure, de reconstituer lesdites liaisons disulfures, en appliquant une composition, généralement oxydante, permettant de fixer la coiffure. Cette technique permet ainsi de réaliser indifféremment soit une ondulation, soit un défrisage, soit un crêpage des cheveux. La nouvelle forme imposée aux cheveux par un traitement chimique tel que décrit ci-dessus est éminemment durable dans le temps et résiste notamment à l'action des lavages à l'eau ou des shampooings, et ceci par opposition aux simples techniques classiques de déformation temporaire, telles que de mise en plis.

Les compositions réductrices pour la déformation permanente des cheveux contiennent dans la plupart des cas, des agents pouvant générer une altération des cheveux déjà sensibilisés ou colorés, malgré la présence d'agents conditionneurs dans ces compositions. L'utilisation de pré-lotion sur ce type de cheveux est nécessaire pour préserver l'intégrité du cheveu en ralentissant la pénétration de l'agent réducteur.
Malheureusement, ces pré-lotions ont pour effet de freiner les performances de la permanente en frisure et en nervosité.

Ainsi, il s'avère nécessaire d'améliorer les techniques de déformation permanente sur des cheveux déjà sensibilisés ou colorés notamment en remédiant aux problèmes ci-dessus.

Plus précisément, il existe un réel besoin de fournir un procédé permettant de réaliser une déformation permanente, qui procure aux cheveux déjà sensibilisés ou colorés des déformations, des boucles ou des frisures nerveuses tout en conservant de bonnes qualités cosmétiques telles que la douceur ou le démêlage et tout en préservant la coloration artificielle des cheveux quand ceux-ci sont déjà colorés.

La demanderesse a découvert qu'il était possible de résoudre les problèmes décrits plus haut en utilisant une composition réductrice comprenant un rapport de concentrations molaires en ammonium défini, une teneur en ammoniaque définie, et un polymère particulier.

Un autre objet de l'invention concerne un procédé pour la déformation permanente des fibres kératiniques et en particulier des cheveux, comprenant l'application de la composition précitée.

Un autre objet de l'invention concerne un dispositif à compartiments
ou kit comprenant au moins une composition réductrice définie ci-dessus.

D'autres objets, caractéristiques, aspects et avantages de l'invention apparaîtront encore plus clairement à la lecture de la description et des divers exemples qui suivent.

Bien que l'exposé qui suit s'articule essentiellement autour du cas particulier du traitement du cheveu, le procédé de l'invention est applicable à toute matière kératinique en général, notamment cils, moustaches, poils et autres.

Les compositions réductrices de l'invention pour la déformation permanente des matières kératiniques et en particulier les cheveux comprennent :

- au moins du bicarbonate d'ammonium et au moins un autre agent alcalin choisi parmi l'ammoniaque et/ou les sels d'ammonium différents du bicarbonate d'ammonium, tel que ces compositions présentent un rapport R de concentrations molaires défini par la formule suivante :

$$R = \frac{\text{nombre de moles d' ammonium total} - \text{nombre de moles de bicarbonate d' ammonium}}{\text{nombre de moles de bicarbonate d' ammonium}}$$

  supérieur à 2,1,
- au moins un polymère cationique non cyclique, c'est-à-dire au moins un polymère contenant des groupements cationiques et/ou des groupements ionisables en groupements cationiques, à l'exception de ceux dont la charge cationique est portée par un atome d'azote inclus dans un cycle, choisi parmi les polymères de type polyammonium quaternaire possédant les motifs récurrents de formule (W) suivante :

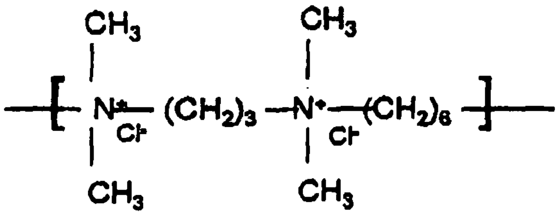

(W)

et dont le poids moléculaire est compris entre 9500 et 9900 ; et
les polyméres de type polyammonium quaternaire aux motifs récurrents de formule (U) suivante :

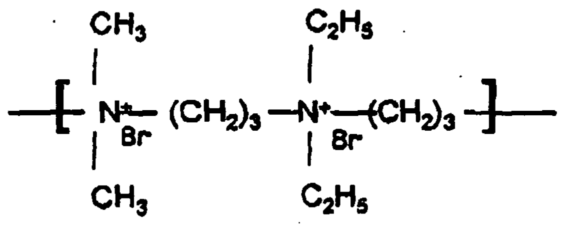

(U)

et dont le poids moléculaire est d'environ 1200,

- lorsque l'ammoniaque est présent, il l'est dans une proportion inférieure à 3% exprimée en $NH_4OH$ en poids par rapport au poids total de la composition,
- et un agent réducteur

De préférence, l'autre agent alcalin est l'ammoniaque.
De préférence, la teneur en ammoniaque est comprise entre 1 et 3%, et de préférence entre 1,5 et 3% en poids par rapport au poids total de la composition.
De préférence, le rapport R est compris entre 2,1 et 5, et plus préférentiellement entre 2,1 et 3.
Dans l'expression R, le nombre de moles d'ammonium total comprend :

- le nombre de moles d'ammonium nécessaire à la neutralisation du thiol
- le nombre de mole d'ammonium en excès
- le nombre de moles de bicarbonate d'ammonium.

Parmi les agents alcalins additionnels ou optionnels, on peut citer la monoéthanolamine, la triéthanolamine, les dérivés de 1,3-diaminopropane.
L'équilibre entre les agents alcalins et l'optimisation de la concentration en bicarbonate d'ammonium de cette composition permet d'améliorer sur des cheveux sensibilisés les performances en qualité de frisure, de nervosité de la boucle et en qualité cosmétique par rapport à des compositions réductrices de l'état de l'art.
Les polymères cationiques non cycliques préférés sont choisis parmi ceux qui contiennent des motifs comportant des groupements amine quaternaire pouvant, soit faire partie de la chaîne principale polymère, soit être portés par un substituant latéral directement relié à celle-ci.
La concentration en polymère cationique non cyclique dans la composition réductrice est comprise, de préférence, entre 0,01 et 20%, et de préférence entre 0,1 et 10% par rapport au poids total de la composition.
Les compositions réductrices de l'invention contiennent au moins un agent réducteur. Cet agent réducteur peut être un sulfite d'un métal alcalin ou alcalino-terreux. De préférence, ce réducteur est un thiol.
Le thiol de la composition réductrice peut être choisi parmi les thiols connus comme agents réducteurs tels que par exemple l'acide thioglycolique, le monothioglycolate de glycérol ou de glycol, la cystéamine et ses dérivés acylés en $C_1$-$C_4$ tels que la N-acétyl cystéamine ou la N-propionyl cystéamine, la cystéine, la N-acétylcystéine, les N-mercaptoalkylamides de sucres tels que le N-(mercapto-2-éthyl)gluconamide, l'acide 3-mercaptopropionique et ses dérivés, l'acide thiolactique et ses esters tel que le monothiolactate de glycérol, l'acide thiomalique, la panthétéine, le thioglycérol, les N-(mercaptoalkyl)-hydroxyalkylamides décrits dans la demande de brevet EP-A-354.835 et les N-mono- ou N,N-dialkylmercapto 4-butyramides décrits dans la demande de brevet EP-A-368.763, les aminomercaptoalkylamides décrits dans la demande de brevet EP-A-432.000, les dérivés des acides N-(mercaptoalkyl) succinamiques ou des N-(mercaptoalkyl) succiminides décrits dans la demande de brevet EP-A-465.342, les alkylaminomercaptoalkylamides décrits dans

la demande de brevet EP-A-514.282, le mélange de thioglycolate d'hydroxy-2-propyle et de thioglycolate d'hydroxy-2-méthyl-1 éthyle décrit dans la demande de brevet FR-A-2.679.448.

On utilise de préférence l'acide thioglycolique, l'acide thiolactique, la cystéine, la cystéamine ou un de leurs sels, ou le thioglycolate de glycérol.

Encore plus préférentiellement, le thiol est l'acide thioglycolique, la cystéine ou l'un de leurs sels.

Le ou les agents réducteurs sont généralement présents à une concentration qui peut être comprise entre 1 et 20 % en poids par rapport au poids total de la composition réductrice.

Le pH de la composition réductrice est, généralement, compris entre 6 et 10 et de préférence entre 7 et 9.

La composition réductrice peut se présenter sous la forme d'une lotion, épaissie ou non, d'une crème, d'un gel ou de tout autre forme appropriée et peut contenir des additifs connus pour leur utilisation dans les compositions réductrices pour la déformation permanente des cheveux.

La composition réductrice peut également contenir un solvant tel que par exemple de l'éthanol, du propanol ou de l'isopropanol ou encore du glycérol à une concentration maximale de 20 % par rapport au poids total de la composition.

Un autre objet de l'invention porte sur un procédé pour la déformation permanente des fibres kératiniques, en particulier des cheveux comprenant les étapes suivantes :

(i) on applique sur la matière kératiniques une composition réductrice telle que définie précédemment,
(ii) après un temps de pose nécessaire à la réduction de la matière kératinique, et un éventuel rinçage, on applique une composition fixatrice,
(iii) après un temps de repos nécessaire à la fixation, on rince la matière kératinique ainsi traitée.

La matière kératinique peut être mise sous tension, notamment à l'aide de rouleaux, de bigoudis ou analogues, avant, pendant ou après l'application de la composition réductrice de l'étape (i).

Une pré-lotion peut être appliquée sur la matière kératinique avant l'application de la composition réductrice. Cette pré-lotion peut contenir une silicone aminée.

La composition réductrice peut être appliquée sur une matière kératinique humide.

Il convient, de manière classique, de laisser reposer pendant quelques minutes, généralement entre 2 et 40 minutes, de préférence entre 5 et 30 minutes, la chevelure sur laquelle a été appliquée la composition réductrice, et ceci de façon à bien laisser le temps au réducteur d'agir correctement sur la matière kératinique. Cette phase d'attente est effectuée généralement en laissant reposer (à température ambiante ou avec chauffage) la chevelure traitée.

Les cheveux imprégnés de la composition réductrice peuvent être, ensuite, rincés soigneusement, généralement à l'eau. Eventuellement, on met en oeuvre une phase de chauffage à température élevée pendant quelques secondes.

Puis, on applique sur les cheveux une composition oxydante dans le but de fixer la nouvelle forme imposée aux cheveux. Il peut également être envisagé de laisser les cheveux s'oxyder à l'air.

Cette composition fixatrice peut contenir tout agent oxydant connu en soi. Avantageusement, la composition fixatrice comprend au moins un agent oxydant choisi parmi le peroxyde d'hydrogène, le peroxyde d'urée, les bromates de métaux alcalins, les persels tels que les perborates et les persulfates.

Le pH de la composition fixatrice est généralement compris entre 2 et 10.

Comme dans le cas de l'application de la composition réductrice, la chevelure sur laquelle a été appliquée la composition fixatrice est ensuite, de manière classique, laissée dans une phase de repos nécessaire à la fixation de la chevelure ou d'attente qui dure quelques minutes, généralement entre 3 et 30 minutes, de préférence entre 5 et 15 minutes.

Dans la dernière étape du procédé selon l'invention (étape (iii)), la chevelure ainsi traitée est rincée après un temps de repos.

On enlève de la chevelure les moyens mécaniques (rouleaux, bigoudis et analogues) qui maintenaient sous tension dans la forme désirée les cheveux tout au long du traitement, avant ou après rinçage de la composition fixatrice, la suppression des moyens de mise sous tension pouvant éventuellement être suivie d'une nouvelle application d'une certaine quantité de composition fixatrice.

Le procédé se termine par un séchage naturel ou par tout autre moyen de séchage (infrarouge, sèche-cheveux et autres), afin d'obtenir une belle frisure.

La composition fixatrice contient également des agents régulateurs de pH, de manière à maintenir un pH adéquat.

Les compositions fixatrice ou réductrice peuvent contenir, en outre, de préférence, des additifs choisis parmi les tensioactifs, les silicones, les cires, les épaississants, les agents de pénétration, les alcools gras, les dérivés de lanoline, les céramides, les ingrédients actifs, les agents anti-chute, anti-pelliculaires, les agents de mise en suspension, les agents séquestrants, les agents opacifiants, les agents stabilisants, les colorants, les filtres solaires siliconés ou

non, les conservateurs, les parfums et les composés dithio-.

On obtient finalement une chevelure présentant de bonnes propriétés cosmétiques : les cheveux sont plus brillants, plus doux et plus faciles à démêler ou à coiffer. Les cheveux présentent de bonnes qualités de frisure et de nervosité de la boucle même sur des cheveux sensibilisés.

Un autre objet de l'invention concerne un dispositif à compartiments ou kit pour la déformation permanente des fibres kératiniques comprenant dans un premier compartiment une composition réductrice telle que définie précédemment, et dans un second compartiment une composition fixatrice.

L'invention pourra être mieux comprise à l'aide des exemples non limitatifs qui suivent et qui constituent des modes de mise en oeuvre avantageux du procédé selon l'invention.

Exemples de compositions

La demanderesse a réalisé une composition réductrice (1) conforme à l'invention :

| | |
|---|---|
| Acide thioglycolique | 7g |
| Sel pentasodique de l'acide diéthylène triaminopentacétique | 0,4g |
| Ammoniaque en solution aqueuse à 20% de $NH_3$ | 6,8g |
| Bicarbonate d'ammonium | 2,5g |
| MEXOMERE PO (CHIMEX)* | 1,6g |
| Eau déminéralisée | qsq 100g |

La demanderesse a réalisé une composition réductrice (2) conforme à l'invention :

| | |
|---|---|
| Acide thioglycolique | 6,7g |
| Sel pentasodique de l'acide diéthylène triaminopentacétique | 0,4g |
| Ammoniaque en solution aqueuse à 20% de $NH_3$ | 6,5g |
| Bicarbonate d'ammonium | 2,5g |
| MEXOMERE PO (CHIMEX)* | 1,6g |
| Eau déminéralisée | qsq 100g |
| * Polymère cationique non cyclique de structure W en solution aqueuse | |

La demanderesse a réalisé la composition fixatrice (3) suivante :

| | |
|---|---|
| Peroxyde d'hydrogène en solution 50% | 4,8g |
| Oxyde de lauryl diméthylamine en solution aqueuse (30%) | 2,15g |
| MERQUAT 100 | 1,25g |
| Acide citrique | 0,1g |
| Eau déminéralisée | qsq 100g |

Résultats d'essais sur tête en comparant avec une permanente Dulcia Vital 2 (DV2) commercialisée par la société L'OREAL

I-Avec la composition réductrice (1)

La composition (1) a été appliquée sur cheveux humides roulés sur bigoudis et posée pendant 10 minutes. De l'autre côté, le réducteur DV2 a été appliqué pendant 10 minutes. Après rinçage, la fixation a été réalisée avec la composition (3) et le fixateur DV2 pendant 5 minutes avant le rinçage final.

Sur l'ensemble des modèles (8), les nouvelles compositions conduisent à un cheveu humide jugé plus doux, plus facile à démêler, avec des boucles plus nerveuses, et sur cheveux séchés à une amélioration du démêlage, de la

douceur et de la brillance.

Dans le temps, la demanderesse a observé une meilleure rémanence de ces effets cosmétiques par rapport à la permanente DV2.

Des superpositions (3 applications) ont été effectuées, et une nette supériorité a été obtenue sur l'ensemble des critères de frisure et de cosmétique par rapport à la permanente DV2.

<u>Résultats d'essais sur tête en comparant avec une permanente Dulcia Vital 2 (DV2) commercialiséee par la société L'OREAL avec une pré-lotion Optimiser Présifon 1 commercialisée par la société L'OREAL</u>

II-Avec la composition réductrice (2)

La composition en acide thioglycolique de la composition (2) est équivalente à celle du réducteur de permanente DV2.

La composition (2) a été appliquée sur cheveux humides roulés sur bigoudis et posée pendant 10 minutes. Dans les autres cas, la demanderesse a appliqué le réducteur la pré-lotion Optimiser Présifon 1 avant enroulage sur bigoudis, puis le réducteur DV2 pendant 10 minutes. Après rinçage, la fixation a été réalisée avec la composition (3) et le fixateur DV2 pendant 5 minutes avant le rinçage final.

Sur l'ensemble des modèles, les nouvelles compositions conduisent à une chevelure plus frisée avec des boucles plus nerveuses, et sur cheveux séchés une amélioration du volume pour des qualités cosmétiques équivalentes.

Dans le temps, une meilleure rémanence de ces effets cosmétiques par rapport à la permanente DV2 a été observée.

Des superpositions (3 applications) ont été effectuées, et la demanderesse a obtenu une nette supériorité sur l'ensemble des critères de frisure et de cosmétique par rapport à la permanente DV2.

<u>Influence de la coloration</u>

Des mèches de cheveux naturels à 90% de blancs ayant subi une coloration FERIA® 7.40 commercialisée par L'ORÉAL puis une série de quatre shampooings, ont ensuite été permanentées avec les compositions réductrices (1), (2) et DV2.

La demanderesse a constaté visuellement un moindre décapage de la couleur avec la composition réductrice (2) par rapport aux compositions réductrices (1) et DV2.

## Revendications

1. Composition réductrice pour la déformation permanente des fibres kératiniques, et en particulier des cheveux comprenant :

   - au moins du bicarbonate d'ammonium et au moins un autre agent alcalin choisi parmi l'ammoniaque et/ou les sels d'ammonium différents du bicarbonate d'ammonium et présentant un rapport de concentrations molaires R défini dans la formule suivante

   $$R = \frac{\text{nombre de moles d' ammonium total} - \text{nombre de moles de bicarbonate d' ammonium}}{\text{nombre de moles de bicarbonate d' ammonium}}$$

   supérieur à 2,1,

   - au moins un polymère cationique noya cyclique, c'est-à-dire au moins un polymère contenant des groupements cationiques et/ou des groupements ionisables en groupements cationiques, à l'exception de ceux dont la charge cationique est portée par un atome d'azote inclus dans un cycle, choisi parmi les polymères de type palyammonium quaternaire possédant les motifs récurrents de formule (W) suivante :

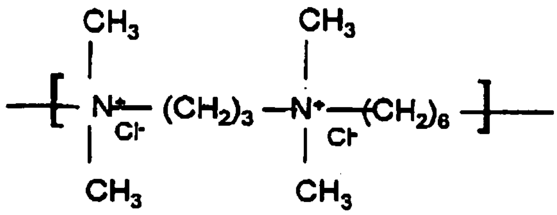

(W)

et dont le poids moléculaire est compris entre 9500 et 9900 ; et
les polymères de type polyammonium quaternaire aux motifs récurrents de formule (U) suivante :

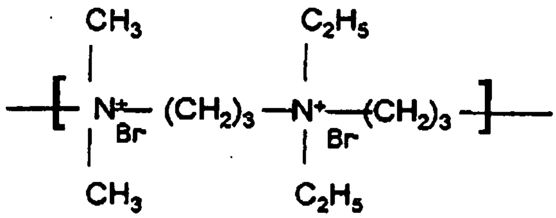

(U)

et dont le poids moléculaire est d'environ 1200,
- lorsque l'ammoniaque est présent, il l'est en proportion inférieure à 3% en poids exprimés en $NH_4OH$ par rapport au poids total de la composition, et au moins un agent réducteur.

2. Composition selon la revendication 1, **caractérisée par le fait que** le rapport R est compris entre 2,1 et 5, et de préférence entre 2,1 et 3 du poids total de la composition.

3. Composition selon l'une quelconque des revendications 1 et 2, **caractérisée par le fait que** l'autre agent alcalin est l'ammoniaque.

4. Composition selon l'une quelconque des revendications 1 à 3, **caractérisée par le fait que** la teneur en ammoniaque est comprise entre 1 et 3%, et de préférence entre 1,5 et 3% du poids total de la composition.

5. Composition selon l'une quelconque des revendications 1 à 4, **caractérisée par le fait que** la composition réductrice contient la monoéthanolamine, la triéthanolamine, les dérivés de 1,3-diaminopropane, comme agents alcalins, additionnels.

6. Composition selon l'une quelconque des revendications 1 à 5, **caractérisée par le fait que** la concentration en polymère cationique non cyclique dans la composition réductrice est comprise entre 0,01 et 20%, et de préférence entre 0,1 et 10% par rapport au poids total de la composition.

7. Composition selon l'une quelconque des revendications 1 à 5, **caractérisée par le fait qu'**elle contient au moins un agent réducteur choisi parmi les sulfites et les thiols.

8. Composition selon la revendication 7, **caractérisé par** le thiol est choisi parmi l'acide thioglycolique, l'acide thiolactique, la cystéine, la cystéamine ou un de leurs sels et le thioglycolate de glycérol.

9. Composition selon l'une quelconque des revendications 1 à 8, **caractérisée par le fait qu'**elle contient des additifs choisis parmi les tensioactifs, les silicones, les cires, les épaississants, les agents de pénétration, les alcools gras, les dérivés de lanoline, les céramides, les ingrédients actifs, les agents anti-chute, anti-pelliculaires, les agents de mise en suspension, les agents séquestrants, les agents opacifiants, les agents stabilisants, les colorants, les filtres solaires siliconés ou non, les conservateurs, les parfums ou des composés dithio.

10. Procédé de déformation permanente des matières kératiniques, et en particulier des cheveux, comprenant les opérations suivantes:

(i) on applique dans un premier temps sur la matière kératinique une composition réductrice telle que définie

dans les revendications 1 à 9,
(ii) après un temps de pose nécessaire à la réduction de la matière kératinique, on applique une composition fixatrice ;
(iii) après un temps de repos nécessaire à la fixation, on rince la matière kératinique ainsi traitée.

**11.** Procédé selon la revendication 10, **caractérisé en ce que** préalablement à l'application de la composition fixatrice, la matière kératinique est rincée.

**12.** Procédé selon la revendication 10 ou 11, **caractérisé par le fait que** la composition fixatrice comprend au moins un agent oxydant choisi parmi le peroxyde d'hydrogène, le peroxyde d'urée, les bromates de métaux alcalins, les persels tels que les perborates et persulfates.

**13.** Procédé selon l'une quelconque des revendications 10 à 12, **caractérisé par le fait qu'**une pré-lotion est appliquée sur la matière kératinique avant l'application de la composition réductrice.

**14.** Procédé selon la revendication 13, **caractérisé par le fait que** la pré-lotion utilisée contient une silicone aminée.

**15.** Procédé selon l'une quelconque des revendications 10 à 14, **caractérisé en ce que** la composition réductrice est appliquée sur une matière kératinique humide.

**16.** Procédé selon l'une quelconque des revendications 10 à 15 **caractérisé par le fait qu'**avant d'appliquer une composition fixatrice on met en oeuvre une phase de chauffage à température élevée pendant quelques secondes.

**17.** Dispositif à compartiments ou kit pour la déformation permanente des fibres kératiniques et en particulier des cheveux, comprenant, dans un premier compartiment une composition réductrice telle que définie dans l'une quelconque des revendications 1 à 9, dans un second compartiment, une composition fixatrice.

## Claims

**1.** Reducing composition for permanently reshaping keratin fibres, and in particular the hair, comprising:

- at least ammonium bicarbonate and at least one other alkaline agent chosen from aqueous ammonia and/or ammonium salts other than ammonium bicarbonate, such that these compositions have a molar concentration ratio R defined by the following formula:

$$R = \frac{\textit{total number of moles of ammonium} - \textit{number of moles of ammonium bicarbonate}}{\textit{number of moles of ammonium bicarbonate}}$$

of greater than 2.1,
- at least one non-cyclic cationic polymer, i.e. at least one polymer containing cationic groups and/or groups that may be ionized into cationic groups, with the exception of those whose cationic charge is borne by a nitrogen atom included in a ring, chosen from polymers of polyquaternary ammonium type containing the repeating units of formula (W) below:

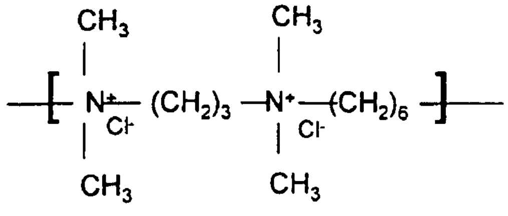
**(W)**

the molecular weight of which is between 9500 and 9900; and
polymers of polyquaternary ammonium type having the repeating units of formula (U) below:

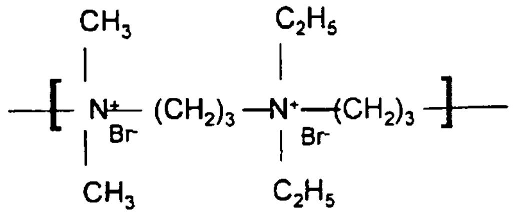

(U)

the molecular weight of which is about 1200,
- when aqueous ammonia is present, it is present in a proportion of less than 3%, expressed as $NH_4OH$, by weight relative to the total weight of the composition, and at least one reducing agent.

**2.** Composition according to Claim 1, **characterized in that** the ratio R is between 2.1 and 5 and preferably between 2.1 and 3 relative to the total weight of the composition.

**3.** Composition according to either of Claims 1 and 2, **characterized in that** the other alkaline agent is aqueous ammonia.

**4.** Composition according to any one of Claims 1 to 3, **characterized in that** the aqueous ammonia content is between 1% and 3% and preferably between 1.5% and 3% relative to the total weight of the composition.

**5.** Composition according to any one of Claims 1 to 4, **characterized in that** the reducing composition contains monoethanolamine, triethanolamine or 1,3-diaminopropane derivatives as additional alkaline agents.

**6.** Composition according to any one of Claims 1 to 5, **characterized in that** the concentration of non-cyclic cationic polymer in the reducing composition is between 0.01% and 20% and preferably between 0.1% and 10% relative to the total weight of the composition.

**7.** Composition according to any one of Claims 1 to 5, **characterized in that** it contains at least one reducing agent chosen from sulfites and thiols.

**8.** Composition according to Claim 7, **characterized in that** the thiol is chosen from thioglycolic acid, thiolactic acid, cysteine and cysteamine or a salt thereof, and glyceryl thioglycolate.

**9.** Composition according to any one of Claims 1 to 8, **characterized in that** it contains additives chosen from surfactants, silicones, waxes, thickeners, penetrating agents, fatty alcohols, lanolin derivatives, ceramides, active ingredients, agents for preventing hair loss, antidandruff agents, suspending agents, sequestering agents, opacifiers, stabilizers, dyes, silicone or non-silicone sunscreens, preserving agents, fragrances or dithio compounds.

**10.** Process for permanently reshaping keratin materials, and in particular the hair, comprising the following operations:

i) in a first stage, a reducing composition as defined in Claims 1 to 9 is applied to the keratin material;
ii) after a leave-in time required for the reduction of the keratin material, a fixing composition is applied;
iii) after a leave-in time required for fixing, the keratin material thus treated is rinsed.

**11.** Process according to Claim 10, **characterized in that**, prior to applying the fixing composition, the keratin material is rinsed.

**12.** Process according to Claim 10 or 11, **characterized in that** the fixing composition comprises at least one oxidizing agent chosen from hydrogen peroxide, urea peroxide, alkali metal bromates and persalts such as perborates and persulfates.

**13.** Process according to any one of Claims 10 to 12, **characterized in that** a prelotion is applied to the keratin material

before applying the reducing composition.

**14.** Process according to Claim 13, **characterized in that** the prelotion used contains an amino silicone.

**15.** Process according to any one of Claims 10 to 14, **characterized in that** the reducing composition is applied to a moistened keratin material.

**16.** Process according to any one of Claims 10 to 15, **characterized in that**, before applying a fixing composition, a phase of heating at high temperature for a few seconds takes place.

**17.** Device with compartments or kit for permanently reshaping keratin fibres, and in particular the hair, comprising a reducing composition as defined in any one of Claims 1 to 9 in a first compartment, and a fixing composition in a second compartment.

## Patentansprüche

**1.** Reduzierende Zusammensetzung zur dauerhaften Verformung von Keratinfasern und insbesondere von Haaren, umfassend:

- mindestens Ammoniumhydrogencarbonat und mindestens ein anderes alkalisches Mittel, das unter Ammoniak und/oder Ammoniumsalzen, die von Ammoniumhydrogencarbonat verschieden sind, ausgewählt ist, mit einem Verhältnis R der molaren Konzentrationen, das in der folgenden Formel definiert ist:

*R = (Gesamtzahl der Mole von Ammonium - Zahl der Mole von Ammoniumhydrogencarbonat)/Zahl der Mole von Ammoniumhydrogencarbonat*

von mehr als 2,1,
- mindestens ein nichtcyclisches kationisches Polymer, d.h. mindestens ein Polymer mit kationischen Gruppen und/oder zu kationischen Gruppen ionisierbaren Gruppen mit Ausnahme derjenigen, deren kationische Ladung von einem in einem Ring enthaltenen Stickstoffatom getragen wird,
ausgewählt unter Polymeren vom Typ quartäres Polyammonium mit Wiederholungseinheiten der folgenden Formel (W):

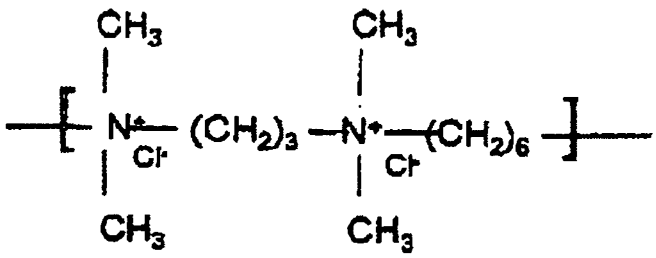

(W)

und einem Molekulargewicht zwischen 9500 und 9900 und
Polymeren vom Typ quartäres Polyammonium mit Wiederholungseinheiten der folgenden Formel (U):

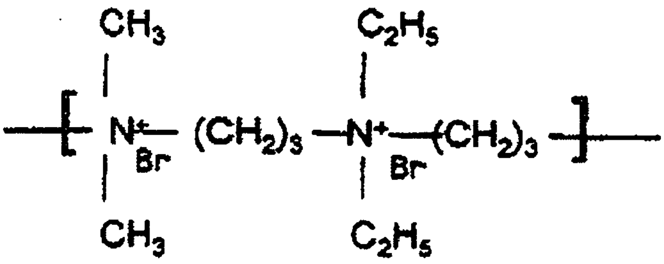

(U)

und einem Molekulargewicht von ungefähr 1200,
- Ammoniak dann, wenn es vorliegt, in einem Anteil von weniger als 3 Gew.-%, ausgedrückt als $NH_4OH$ bezogen auf das Gesamtgewicht der Zusammensetzung, vorliegt, und mindestens ein Reduktionsmittel.

**2.** Zusammensetzung nach Anspruch 1, **dadurch gekennzeichnet, daß** das Verhältnis R zwischen 2,1 und 5 und vorzugsweise zwischen 2,1 und 3, bezogen auf das Gesamtgewicht der Zusammensetzung, beträgt.

**3.** Zusammensetzung nach einem der Ansprüche 1 und 2, **dadurch gekennzeichnet, daß** es sich bei dem anderen alkalischen Mittel um Ammoniak handelt.

**4.** Zusammensetzung nach einem der Ansprüche 1 bis 3, **dadurch gekennzeichnet, daß** der Gehalt an Ammoniak zwischen 1 und 3% und vorzugsweise zwischen 1,5 und 3%, bezogen auf das Gesamtgewicht der Zusammensetzung, beträgt.

**5.** Zusammensetzung nach einem der Ansprüche 1 bis 4, **dadurch gekennzeichnet, daß** die reduzierende Zusammensetzung als zusätzliche alkalische Mittel Monoethanolamin, Triethanolamin oder 1,3-Diaminopropanderivate enthält.

**6.** Zusammensetzung nach einem der Ansprüche 1 bis 5, **dadurch gekennzeichnet, daß** die Konzentration an nichtcyclischem kationischem Polymer in der reduzierenden Zusammensetzung zwischen 0,01 und 20% und vorzugsweise zwischen 0,1 und 10%, bezogen auf das Gesamtgewicht der Zusammensetzung, beträgt.

**7.** Zusammensetzung nach einem der Ansprüche 1 bis 5, **dadurch gekennzeichnet, daß** sie mindestens ein unter Sulfiten und Thiolen ausgewähltes Reduktionsmittel enthält.

**8.** Zusammensetzung nach Anspruch 7, **dadurch gekennzeichnet, daß** das Thiol unter Thioglykolsäure, Thiomilchsäure, Cystein, Cysteamin oder einem Salz davon und Glycerylthioglykolat ausgewählt ist.

**9.** Zusammensetzung nach einem der Ansprüche 1 bis 8, **dadurch gekennzeichnet, daß** sie unter Tensiden, Silikonen, Wachsen, Verdickungsmitteln, Penetriermitteln, Fettalkoholen, Lanolinderivaten, Ceramiden, Wirkstoffen, Mitteln gegen Haarausfall, Mitteln gegen Schuppen, Suspendiermitteln, Sequestriermitteln, Trübungsmitteln, Stabilisatoren, Farbmitteln, Silikon- oder Nichtsilikon-Lichtschutzmitteln, Konservierungsmitteln, Parfümen oder Dithioverbindungen ausgewählte Additive enthält.

**10.** Verfahren zur dauerhaften Verformung von Keratinmaterialien und insbesondere von Haaren, bei dem man:

(i) in einem ersten Schritt auf das Keratinmaterial eine reduzierende Zusammensetzung gemäß einem der Ansprüche 1 bis 9 aufbringt;
(ii) nach einer zur Reduktion des Keratinmaterials notwendigen Einwirkungszeit eine Fixierungszusammensetzung aufbringt;
(iii) nach einer zur Fixierung notwendigen Einwirkungszeit das so behandelte Keratinmaterial spült.

**11.** Verfahren nach Anspruch 10, **dadurch gekennzeichnet, daß** man das Keratinmaterial vor dem Aufbringen der Fixierungszusammensetzung spült.

**12.** Verfahren nach Anspruch 10 oder 11, **dadurch gekennzeichnet, daß** die Fixierungszusammensetzung mindestens

ein unter Wasserstoffperoxid, Harnstoffperoxid, Alkalimetallbromaten und Persalzen wie Perboraten oder Persulfaten ausgewähltes Oxidationsmittel umfaßt.

**13.** Verfahren nach einem der Ansprüche 10 bis 12, **dadurch gekennzeichnet, daß** man vor dem Aufbringen der reduzierenden Zusammensetzung eine Vorlotion auf das Keratinmaterial aufbringt.

**14.** Verfahren nach Anspruch 13, **dadurch gekennzeichnet, daß** die verwendete Vorlotion ein Aminosilikon enthält.

**15.** Verfahren nach einem der Ansprüche 10 bis 14, **dadurch gekennzeichnet, daß** man die reduzierende Zusammensetzung auf ein feuchtes Keratinmaterial aufbringt.

**16.** Verfahren nach einem der Ansprüche 10 bis 15, **dadurch gekennzeichnet, daß** man vor dem Aufbringen einer Fixierungszusammensetzung eine Phase des Erwärmens auf eine hohe Temperatur über einen Zeitraum von einigen Sekunden durchführt.

**17.** Vorrichtung mit Kompartimenten oder Kit zur dauerhaften Verformung von Keratinfasern und insbesondere von Haaren, umfassend in einem ersten Kompartiment eine reduzierende Zusammensetzung gemäß einem der Ansprüche 1 bis 9 und in einem zweiten Kompartiment eine Fixierungszusammensetzung.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 354835 A
- EP 368763 A
- EP 432000 A
- EP 465342 A
- EP 514282 A
- FR 2679448 A